(19) Europäisches Patentamt
European Patent Office
Office européen des brevets

(11) **EP 1 762 621 A1**

(12) **EUROPEAN PATENT APPLICATION**
published in accordance with Art. 158(3) EPC

(43) Date of publication:
**14.03.2007 Bulletin 2007/11**

(51) Int Cl.:
*C12N 15/53* (2006.01)   *C12N 1/21* (2006.01)
*C12N 9/04* (2006.01)   *C12P 7/18* (2006.01)

(21) Application number: **05751178.4**

(22) Date of filing: **15.06.2005**

(86) International application number:
**PCT/JP2005/010960**

(87) International publication number:
**WO 2005/123921 (29.12.2005 Gazette 2005/52)**

(84) Designated Contracting States:
**AT BE BG CH CY CZ DE DK EE ES FI FR GB GR HU IE IS IT LI LT LU MC NL PL PT RO SE SI SK TR**

(30) Priority: **21.06.2004  JP 2004182926**

(71) Applicant: **Kaneka Corporation**
**Kita-ku**
**Osaka-shi, Osaka 530-8288 (JP)**

(72) Inventors:
• **MORIYAMA, Daisuke,**
  **c/o KANEKA CORPORATION**
  **Takasago-shi,**
  **Hyogo 6768688 (JP)**
• **TAOKA, Naoaki,**
  **c/o KANEKA CORPORATION**
  **Takasago-shi,**
  **Hyogo 6768688 (JP)**

(74) Representative: **Vossius & Partner**
**Siebertstrasse 4**
**81675 München (DE)**

(54) **NOVEL GLYCEROL DEHYDROGENASE, GENE THEREFOR, AND METHOD OF UTILIZING THE SAME**

(57)  The present invention provides a polypeptide having physicochemical characteristics of (1) to (5) described below:
(1) action: to generate (S) -3-chloro-1, 2-propanediol by stereoselectively reducing 1-chloro-3-hydroxyacetone using NADH as a coenzyme;
(2) molecular weight: about 340,000 by gel-filtration and about 43,000 by SDS polyacrylamide gel electrophoresis;
(3) optimum temperature: from 60 to 70°C;
(4) optimum pH for reduction: 6.0; and
(5) optimum pH for oxidation: 9.0.

Furthermore, the present invention provides a polypeptide comprising the amino acid sequence represented by SEQ ID NO: 1 in the Sequence Listing, a DNA encoding the polypeptide, and a transformant producing the polypeptide in large quantities. Still furthermore, the present invention provides a manufacturing method by using the above polypeptide or the above transformant of (S)-3-chloro-1,2-propanediol that is a useful material for pharmaceuticals, etc.

EP 1 762 621 A1

SEQUENCE LISTING

<110> KANEKA CORPORATION

<120> NOVEL GLYCEROL DEHYDROGENASE, GENE THEREFOR, AND METHOD OF UTILIZING THE SAME

<130> B040280W001-

<150> JP2004-182926
<151> 2004-06-21

<160> 12

<170> PatentIn version 3.1

<210> 1
<211> 381
<212> PRT
<213> Cellulomonas sp.

<400> 1

Met Ser Glu Val Pro Val Arg Thr Leu Ile Ser Pro Leu Arg Tyr Val
1               5                   10                  15

Gln Ala Arg Gly Ala Leu Thr Arg Leu Gly Glu Phe Val Arg Pro Ile
            20                  25                  30

Gly Thr Lys Pro Leu Leu Val Ala Asp Asp Val Val Trp Gly Ile Val
            35                  40                  45

Gln Asp Thr Val Ala Val Ser Phe Gln Glu Gln Asp Leu Pro Arg His
        50                  55                  60

Arg Thr Gly Phe Gly Thr Tyr Ala Thr Ala Ala Glu Val Asp Arg Leu
65                  70                  75                  80

Ala Arg Glu Ile Asp Glu Leu Gly Ala Asp Val Ile Val Gly Ile Gly
                85                  90                  95

Gly Gly Ser Thr Ile Asp Ala Val Lys Ala Ala Gly His Leu Arg Gly
                100                 105                 110

Ile Arg Trp Val Ser Val Pro Thr Val Ala Ser Thr Asp Ala Pro Cys
            115                 120                 125

Ser Ala Leu Ser Val Ile Tyr Thr Glu Asp Gly Ala Phe Glu Glu Tyr
        130                 135                 140

Arg Phe Phe Pro His Asn Pro Asp Leu Val Leu Val Asp Thr Gly Leu
145                 150                 155                 160

Val Ala Asn Ala Pro Val Lys Phe Leu Val Ala Gly Val Gly Asp Ala
                165                 170                 175

Leu Ala Thr Trp Ile Glu Ala Arg Ala Val Ala Glu Ala Asn Ala Ser
                180                 185                 190

Thr Met Ala Gly Gly Leu Pro Leu Val Thr Gly Thr Ala Leu Ala Gln
            195                 200                 205

Leu Ser Trp Asp Ile Leu Trp Glu Asn Ala Leu Pro Ala Ile Asp Ala
        210                 215                 220

Val Lys Asn His Leu Val Thr Pro Ala Val Glu Lys Val Val Glu Ala

14

```
                 225                  230                  235                  240

        Asn Thr Leu Leu Ser Gly Leu Gly Phe Glu Ser Gly Gly Leu Ala Ala
                        245                  250                  255

        Ala His Ala Ile His Asn Gly Leu Thr Ala Ala Pro Gln Thr His Gly
                    260                  265                  270

        Leu Thr His Gly Gln Lys Val Asn Ile Gly Ser Val Thr Gln Leu Val
                    275                  280                  285

        Leu Glu Gly Ala Pro Thr Glu Glu Ile Glu Glu Phe Val Arg Phe Thr
            290                  295                  300

        Thr Arg Val Gly Leu Pro Asn Thr Leu Thr Glu Ile Gly Leu Thr Ala
        305                  310                  315                  320

        Asp Asp Val His Asp Leu Thr Arg Val Ala Glu Ala Ala Thr Ala Glu
                        325                  330                  335

        Gly Glu Thr Ile His Ala Met Pro Phe Pro Val Arg Val Pro Glu Leu
                        340                  345                  350

        Val Asp Ala Leu Arg Ser Ile Glu Gly Phe Ser Arg Arg Val Arg Ala
                        355                  360                  365

        Glu Ala Gly Leu Pro Glu Pro Val Lys His Thr Ala His
                    370                  375                  380


        <210> 2
        <211> 1146
        <212> DNA
        <213> Cellulomonas sp.

        <220>
        <221> CDS
        <222> (1)..(1143)
        <223>

        <400> 2
        atg tcc gag gtt ccc gtc cgc acg ctc atc agc ccg ctg cgc tac gtc      48
        Met Ser Glu Val Pro Val Arg Thr Leu Ile Ser Pro Leu Arg Tyr Val
        1               5                   10                  15

        cag gcc cgc ggt gcg ctc acc cgc ctc ggc gag ttc gtc cgg ccg atc      96
        Gln Ala Arg Gly Ala Leu Thr Arg Leu Gly Glu Phe Val Arg Pro Ile
                    20                  25                  30

        ggc acg aag ccg ctg ctc gtc gcc gac gac gtc gtg tgg ggc atc gtc     144
        Gly Thr Lys Pro Leu Leu Val Ala Asp Asp Val Val Trp Gly Ile Val
                35                  40                  45

        cag gac acc gtc gcc gtg tcc ttc cag gag cag gac ctc ccc cgc cac     192
        Gln Asp Thr Val Ala Val Ser Phe Gln Glu Gln Asp Leu Pro Arg His
            50                  55                  60

        cgc acc ggc ttc ggc acg tac gcg acc gcc gcc gag gtc gac cgg ctc     240
        Arg Thr Gly Phe Gly Thr Tyr Ala Thr Ala Ala Glu Val Asp Arg Leu
        65                  70                  75                  80

        gcg cgc gag atc gac gag ctc ggc gct gac gtg atc gtc ggc atc ggc     288
        Ala Arg Glu Ile Asp Glu Leu Gly Ala Asp Val Ile Val Gly Ile Gly
                        85                  90                  95

        ggc ggc tcg acc atc gac gcg gtc aag gcc gcg ggc cac ctg cgc ggc     336
        Gly Gly Ser Thr Ile Asp Ala Val Lys Ala Ala Gly His Leu Arg Gly
                    100                 105                 110

        atc cgc tgg gtc tcc gtg ccg acc gtc gcg tcc acc gac gcc ccg tgc     384
        Ile Arg Trp Val Ser Val Pro Thr Val Ala Ser Thr Asp Ala Pro Cys
```

```
                  115                   120                   125
          tcc gcg ctg tcc gtc atc tac acc gag gac ggc gcc ttc gag gag tac      432
          Ser Ala Leu Ser Val Ile Tyr Thr Glu Asp Gly Ala Phe Glu Glu Tyr
              130                   135                   140

          cgg ttc ttc ccg cac aac ccg gac ctc gtg ctc gtc gac acg ggc ctc      480
          Arg Phe Phe Pro His Asn Pro Asp Leu Val Leu Val Asp Thr Gly Leu
          145                   150                   155                   160

          gtc gcg aac gcg ccc gtg aag ttc ctc gtc gcc ggc gtc ggt gac gcg      528
          Val Ala Asn Ala Pro Val Lys Phe Leu Val Ala Gly Val Gly Asp Ala
                              165                   170                   175

          ctc gcg acg tgg atc gag gcg cgc gcc gtc gcc gag gcg aac gcg tcg      576
          Leu Ala Thr Trp Ile Glu Ala Arg Ala Val Ala Glu Ala Asn Ala Ser
                          180                   185                   190

          acc atg gcc ggt ggc ctc ccc ctc gtc acg ggc acg gcg ctc gcg cag      624
          Thr Met Ala Gly Gly Leu Pro Leu Val Thr Gly Thr Ala Leu Ala Gln
                      195                   200                   205

          ctc tcg tgg gac atc ctc tgg gag aac gcg ctg ccc gcg atc gac gcg      672
          Leu Ser Trp Asp Ile Leu Trp Glu Asn Ala Leu Pro Ala Ile Asp Ala
              210                   215                   220

          gtg aag aac cac ctc gtg acg ccc gcg gtc gag aag gtc gtc gag gcg      720
          Val Lys Asn His Leu Val Thr Pro Ala Val Glu Lys Val Val Glu Ala
          225                   230                   235                   240

          aac acg ctc ctg tcc ggg ctg ggc ttc gag tcc ggc ggc ctc gcg gcg      768
          Asn Thr Leu Leu Ser Gly Leu Gly Phe Glu Ser Gly Gly Leu Ala Ala
                              245                   250                   255

          gcg cac gcc atc cac aac ggc ctc acc gcc gcg ccc cag acg cac ggg      816
          Ala His Ala Ile His Asn Gly Leu Thr Ala Ala Pro Gln Thr His Gly
                          260                   265                   270

          ctc acg cac ggc cag aag gtg aac atc ggc tcc gtg acg cag ctc gtg      864
          Leu Thr His Gly Gln Lys Val Asn Ile Gly Ser Val Thr Gln Leu Val
                      275                   280                   285

          ctg gag ggc gcg ccg acc gag gag atc gag gag ttc gtg cgc ttc acg      912
          Leu Glu Gly Ala Pro Thr Glu Glu Ile Glu Glu Phe Val Arg Phe Thr
              290                   295                   300

          acg cgc gtc ggc ctg ccg aac acg ctc acc gag atc ggc ctg acc gcc      960
          Thr Arg Val Gly Leu Pro Asn Thr Leu Thr Glu Ile Gly Leu Thr Ala
          305                   310                   315                   320

          gac gac gtg cac gac ctc acg cgc gtc gcg gaa gcc gcc acc gcg gag     1008
          Asp Asp Val His Asp Leu Thr Arg Val Ala Glu Ala Ala Thr Ala Glu
                              325                   330                   335

          ggc gag acg atc cac gcc atg ccg ttc ccc gtg cgc gtg ccc gag ctc     1056
          Gly Glu Thr Ile His Ala Met Pro Phe Pro Val Arg Val Pro Glu Leu
                          340                   345                   350

          gtg gac gcg ctg cgc tcc atc gag ggc ttt tcc cgc cgc gtc cgc gcc     1104
          Val Asp Ala Leu Arg Ser Ile Glu Gly Phe Ser Arg Arg Val Arg Ala
                      355                   360                   365

          gag gcc ggc ctc ccc gag ccg gtc aag cac acc gcc cac tga            1146
          Glu Ala Gly Leu Pro Glu Pro Val Lys His Thr Ala His
              370                   375                   380
```

```
<210>  3
<211>  20
<212>  DNA
<213>  Artificial

<220>
<223>  primer-1

<220>
<221>  misc_feature
<222>  (3)..(3)
<223>  n represents a, t, g or c
```

```
<220>
<221>  misc_feature
<222>  (12)..(12)
<223>  n represents a, t, g or c


<220>
<221>  misc_feature
<222>  (15)..(15)
<223>  n represents a, t, g or c


<400>  3
acngaygarg gngmnttyga                                    20


<210>  4
<211>  20
<212>  DNA
<213>  Artificial

<220>
<223>  primer-2

<220>
<221>  misc_feature
<222>  (6)..(6)
<223>  n represents a, t, g or c


<220>
<221>  misc_feature
<222>  (15)..(15)
<223>  n represents a, t, g or c


<400>  4
ggcatntkrt gdatngtytc                                    20


<210>  5
<211>  24
<212>  DNA
<213>  Artificial

<220>
<223>  primer-3

<400>  5
ttctcccaga ggatgtccca cgag                               24


<210>  6
<211>  24
<212>  DNA
<213>  Artificial

<220>
<223>  primer-4

<400>  6
agatcgagga gttcgtgcgc ttca                               24


<210>  7
<211>  27
<212>  DNA
<213>  Artificial

<220>
<223>  primer-5

<400>  7
gatcatatgt ccgaggttcc cgtccgc                            27


<210>  8
<211>  33
<212>  DNA
```

```
<213> Artificial

<220>
<223> primer-6

<400> 8
ctagaattct tatcagtggg cggtgtgctt gac                    33


<210> 9
<211> 43
<212> DNA
<213> Artificial

<220>
<223> primer-7

<400> 9
gccgaattct aaggaggtta acaatgtccg aggttcccgt ccg          43


<210> 10
<211> 32
<212> DNA
<213> Artificial

<220>
<223> primer-8

<400> 10
gcgggatcct tatcagtggg cggtgtgctt ga                     32


<210> 11
<211> 43
<212> DNA
<213> Artificial

<220>
<223> primer-9

<400> 11
gccggatcct aaggaggtta acaatgtata aadatttaga agg          43


<210> 12
<211> 29
<212> DNA
<213> Artificial

<220>
<223> primer-10

<400> 12
gcgctgcagt tatccgcgtc ctgcttgga                         29
```

**Description**

Technical Field

The present invention relates to a novel polypeptide, a gene encoding the polypeptide, a vector comprising the gene, a transformant comprising the vector, and a manufacturing method of optically active alcohols by utilizing the transformant.

More specifically, the present invention relates to glycerol dehydrogenase that is isolated from microorganisms having an enzyme activity to stereoselectively reduce 1-chloro-3-hydroxyacetone to generate (S)-3-chloro-1,2-propanediol and that has the above enzyme activity; a DNA encoding the enzyme; an expression vector comprising the DNA; and a transformant transformed by the expression vector. The present invention also relates to a manufacturing method of (S)-3-chloro-1,2-propanediol that is a useful intermediate for pharmaceuticals, etc.

Glycerol dehydrogenase is an enzyme that catalyzes a reaction generating dihydroxyacetone from glycerol using oxidized nicotinamide adenine dinucleotide (hereinafter referred to as NAD+) or oxidized nicotinamide adenine dinucleotide phosphate (hereinafter referred to as NADP) as a coenzyme thereof, and a reaction stereoselectively reducing a carbonyl group of α-hydroxyketones using reduced nicotinamide adenine dinucleotide (hereinafter referred to as NADH) or reduced nicotinamide adenine dinucleotide phosphate (hereinafter referred to as NADPH) as a coenzyme thereof.

Glycerol dehydrogenase, which not only can be utilized for quantitative determination of glycerol, etc., but also can be used as a catalyst for production of optically active 1,2-diols that are useful as raw materials for synthesis of pharmaceuticals and the like, is an industrially useful enzyme.

Background Art

Glycerol dehydrogenase is known to be present in green algae, yeasts, molds, bacteria, ray fungi, etc. Specifically, enzymes derived from *Candida valida* (Non-patent Document 1) , *Schizosaccharomyces pombe* (Non-patent Document 2), *Aspergillus niger* (Non-patent Document 3), *Aspergillus nidulans* (Non-patent Document 3), *Bacillus stearothermophilus* (Non-patent Document 4) , *Cellulomonas* sp. NT3060 (Non-patent Document 5) , *Escherichia coli* (Non-patent Document 6) , and *Lactobacillus fermentum* (Patent Document 1), etc., have been reported.

Among these, the entire amino acid sequences are known for glycerol dehydrogenase derived from *Schizosaccharomyces pombe, Bacillus stearothermophilus,* and *Escherichia coli,* which, however, are apparently different from the amino acid sequence of the enzyme of the present invention.

On the other hand, as for glycerol dehydrogenase derived from microorganisms of the genus *Cellulomonas,* one N-terminal amino acid and two C-terminal amino acids of the enzyme derived from the above-described *Cellulomonas* sp. NT3060 have been determined but its entire amino acid sequence has not been reported.

Furthermore, for a method for manufacturing (S)-3-chloro-1,2-propanediol, a method in which 1-chloro-3-hydroxyacetone is stereoselectively reduced using glycerol dehydrogenase derived from *Cellulomonas* sp. from SIGMA (Patent Document 2) or recombinant E. *coli* having a glycerol dehydrogenase gene derived from *Serratia marcescens* IFO12468 transferred therein (Patent Document 3) is known. However, in terms of industrial applications, there are great disadvantages such that in the former process the expensive commercial enzyme is required, and in the latter process the stability against the substrate, 1-chloro-3-hydroxyacetone, is not high so that a concentration of the substrate to be charged and a conversion rate from the substrate to the product are low.

[Patent Document 1] Japanese Patent Laid-Open No. 10-113170
[Patent Document 2] WO03/018523
[Patent Document 3] Japanese Patent Laid-Open No. 2003-61668
[Non-patent Document 1] J. Gen. Microbiol., 130, 3225 (1984)
[Non-patent Document 2] J. Gen. Microbiol., 131, 1581 (1985)
[Non-patent Document 3] J. Gen. Microbiol., 136, 1043 (1990)
[Non-patent Document 4] Biochim. Biochem. Acta, 994, 270 (1989)
[Non-patent Document 5] Agric. Biol. Chem., 48, 1603 (1982)
[Non-patent Document 6] J. Bacteriol. , 140, 182 (1979)

Disclosure of the Invention

An object of the present invention is to provide a polypeptide that can stereoselectively reduce 1-chloro-3-hydroxyacetone to generate (S)-3-chloro-1,2-propanediol. Another object of the present invention is to efficiently produce the above polypeptide by utilizing recombinant DNA technology. Still another object of the present invention is to provide a practicable manufacturing method of (S)-3-chloro-1,2-propanediol by using the transformant.

We have found a novel polypeptide that can stereoselectively reduce 1-chloro-3-hydroxyacetone to generate (S)-3-chloro-1,2-propanediol. In addition, we have found that (S)-3-chloro-1,2-propanediol can be efficiently manufactured by using the polypeptide or a transformant that can produce the polypeptide. Thus, the present invention has been completed.

Particularly, the present invention is a polypeptide havingphysicochemical characteristics of (1) to (5) described below:

(1) action: to generate (S)-3-chloro-1,2-propanediol by stereoselectively reducing 1-chloro-3-hydroxyacetone using NADH as a coenzyme;
(2) molecular weight: about 340,000 by gel-filtration and about 43,000 by SDS polyacrylamide gel electrophoresis;
(3) optimum temperature: from 60 to 70°C;
(4) optimum pH for reduction: 6.0; and
(5) optimum pH for oxidation: 9.0.

Furthermore, the present invention is the polypeptide (a) or (b) described below:

(a) a polypeptide comprising an amino acid sequence represented by SEQ ID NO: 1 in the Sequence Listing; or
(b) a polypeptide comprising an amino acid sequence in which one or more amino acids are substituted, inserted, deleted, and/or added in the amino acid sequence represented by SEQ ID NO: 1 in the Sequence Listing and having an enzyme activity to stereoselectively reduce 1-chloro-3-hydroxyacetone to generate (S)-3-chloro-1,2-propanediol.

Still furthermore, the present invention is a DNA encoding these polypeptides.

Moreover, the present invention is DNA that hybridizes with DNA comprising a nucleotide sequence complementary to a nucleotide sequence represented by SEQ ID NO: 2 in the Sequence Listing under stringent conditions and that encodes a polypeptide having an enzyme activity to stereoselectively reduce 1-chloro-3-hydroxyacetone to generate (S)-3-chloro-1,2-propanediol.

Still moreover, the present invention is DNA that has at least 60% sequence identity with the nucleotide sequence represented by SEQ ID NO: 2 in the Sequence Listing and that encodes a polypeptide having an enzyme activity to stereoselectively reduce 1-chloro-3-hydroxyacetone to generate (S)-3-chloro-1,2-propanediol.

In addition, the present invention is an expression vector comprising such a DNA and a transformant comprising the expression vector.

Also, the present invention is a manufacturing method of an optically active alcohol comprising the steps of reacting the above polypeptide or a culture of the above transformant with a compound having a carbonyl group and collecting the resulting optically active alcohol.

The present invention provides a novel glycerol dehydrogenase, DNA encoding the enzyme, and a transformant having the DNA. Furthermore, by using the enzyme or the transformant, optically active alcohols, in particular, (S)-3-chloro-1,2-propanediol, can be efficiently made from compounds having a carbonyl group therein.

Brief Description of the Drawings

Figure 1 shows the nucleotide sequence of a gene encoding RCG and the putative amino acid sequence of RCG;
Figure 2 shows construction of recombinant vectors, pTSCS and pTSCSG1; and
Figure 3 shows stability of RCG against 1-chloro-3-hydroxyacetone and 3-chloro-1,2-propanediol.

Best Mode for Carrying Out the Invention

The present invention will be explained in detail below.

The polypeptide of the present invention can be obtained from microorganisms having an activity to stereoselectively reduce 1-chloro-3-hydroxyacetone to generate (S)-3-chloro-1,2-propanediol. Microorganisms used as sources providing the polypeptide include, for example, *Cellulomonas* sp. strain KNK0102. The above strain KNK0102 is a microorganism isolated from the soil by the present inventors.

Mycological properties of *Cellulomonas* sp. strain KNK0102 are described below.

A. Morphology

(1) A cell has a size of 0.5 to 0.7 $\mu$m by 1.5 to 2.0 $\mu$m and an irregular rod shape.
(2) A cell is motile, having one flagellum.
(3) No spore.
(4) Gram stain: positive.
(5) Colony morphology: circular, convex, entire, and yellow.

B. Physiological properties

(1) Hydrolysis of gelatin: positive.
(2) Hydrolysis of starch: positive.
(3) Reduction of nitrate: positive.
(4) Catalase activity: positive.
(5) Oxidase activity: negative.
(6) Urease activity: negative.
(7) DNAse activity: positive.
(8) Aminopeptidase activity: negative.
(9) Cellulose decomposition: negative.
(10) The Voges-Proskauer test: negative.
(11) The methyl red test: negative.
(12) The O-F test: fermentation.
(13) Decomposition of carbohydrates: in anaerobic conditions, acids are generated from fructose, galactose, glucose, glycogen, maltose, starch, sucrose, and xylose, but not from glycerin, lactose, mannitol, and mannose.

A microorganism producing the polypeptide of the present invention can be either a wild strain or a variant. Furthermore, a microorganism derived by a genetic technique such as cell fusion or gene manipulation can be used. A microorganism genetically manipulated to produce the polypeptide of the present invention can be obtained, for example, by the method comprising the steps of: isolating and/or purifying such an enzyme and determining a partial or entire amino acid sequence of the enzyme; determining a nucleotide sequence of DNA encoding the polypeptide based on an amino acid sequence thereof; obtaining DNA encoding the polypeptide based on the nucleotide sequence; obtaining a recombinant microorganism by transferring the DNA into a suitable host microorganism; and obtaining the enzyme of the present invention by culturing the recombinant microorganism.

The polypeptides of the present invention may include, for example, a polypeptide having physicochemical characteristics of (1) to (5) described below:

(1) action: to generate (S) -3-chloro-1, 2-propanediol by stereoselectively reducing 1-chloro-3-hydroxyacetone using NADH as a coenzyme;
(2) molecular weight: about 340,000 by gel-filtration and about 43,000 by SDS polyacrylamide gel electrophoresis;
(3) optimum temperature: from 60 to 70°C;
(4) optimum pH for reduction: 6.0; and
(5) optimum pH for oxidation: 9.0.

The reduction activity of the enzyme can be determined, for example, by adding 20 mM of the substrate, 0.167 mM of coenzyme NADH, and the enzyme solution to a 100 mM phosphate buffer (pH 6.5) followed by the measurement of a decrease in absorbance at 340 nm at 30°C.

The oxidation activity of the enzyme can be determined, for example, by adding 0.1 M of the substrate, 2 mM of coenzyme $NAD^+$, and the enzyme solution to a 100 mM phosphate buffer (pH 8.0) followed by the measurement of an increase in absorbance at 340 nm at 30°C.

The molecular weight of the enzyme can be determined, for example, by calculating from relative elution times of standard proteins on gel-filtration using a Superdex 200HR10/30 column (fromPharmaciaBiotech) . Furthermore, the molecular weight of the subunit can be determined by calculating from relative mobilities of standard proteins on 20% SDS-polyacrylamide gel electrophoresis.

The optimum pH and temperature of the enzyme can be determined, for example, by measuring activities by varying reaction pH and temperature in the system described above to determine the reduction activity.

The polypeptide of the present invention has resistance to 1-chloro-3-hydroxyacetone and 3-chloro-1,2-propanediol. That is, in the reaction to generate (S)-3-chloro-1,2-propanediol by stereoselectively reducing 1-chloro-3-hydroxyacetone, the polypeptide of the present invention is not readily subject to substrate inhibition and/or product inhibition. Therefore, the polypeptide of the present invention is suitable in manufacturing (S)-3-chloro-1,2-propanediol.

Herein, the expression "having resistance to 1-chloro-3-hydroxyacetone" means that when 1-chloro-3-hydroxyacetone is added to a solution containing the polypeptide (the enzyme) of the present invention at the final concentration of 0.25% and the mixture is incubated at 30°C for 20 hrs. followed by the determination of the activity to reduce 1-chloro-3-hydroxyacetone under the conditions described above, the resulting activity is 80% or more, preferably 85% or more, more preferably 90% or more of the activity determined without the pretreatment.

Likewise, the expression "having resistance to 3-chloro-1,2-propanediol" means that when the activity to reduce 1-chloro-3-hydroxyacetone is determined after incubation at 30°C for 20 hrs. in the presence of 1% of 3-chloro-1,2-propanediol, the resulting activity is 80% or more, preferably 90% or more, more preferably 95% or more of the activity determined without the pretreatment.

Furthermore, the polypeptide of the present invention may be a polypeptide comprising an amino acid sequence represented by SEQ ID NO: 1 in the Sequence Listing; or a polypeptide comprising an amino acid sequence in which one or more amino acids are substituted, inserted, deleted, and/or added in the amino acid sequence represented by SEQ ID NO: 1 in the Sequence Listing and having an enzyme activity to stereoselectively reduce 1-chloro-3-hydroxyacetone to generate (S)-3-chloro-1,2-propanediol.

The polypeptide comprising an amino acid sequence in which one or more amino acids are substituted, inserted, deleted, and/or added in the amino acid sequence represented by SEQ ID NO: 1 in the Sequence Listing can be prepared according to the well-known method described in Current Protocols in MolecularBiology (JohnWileyandSons, Inc. , 1989) , etc. Such a polypeptide is included by the polypeptide in the present invention, as long as such a polypeptide has an enzyme activity to stereoselectively reduce 1-chloro-3-hydroxyacetone to generate (S)-3-chloro-1,2-propanediol.

The position where amino acids are substituted, inserted, deleted, and/or added in the amino acid sequence represented by SEQ ID NO: 1 in the Sequence Listing is not particularly limited, but preferably highly conserved regions are avoided. Herein, a highly conserved region refers to the region where amino acids are identical among a plurality of sequences, when the amino acid sequences of a plurality of dehydrogenases of different origins are optimally aligned and compared. A highly conserved region can be confirmed by comparing using a tool such as GENETYX the amino acid sequence represented by SEQ ID NO: 1 with the amino acid sequences of other glycerol dehydrogenases derived from *Bacillus stearothermophilus* and *Escherichia coli* described above.

An amino acid sequence modified by substitution, insertion, deletion, and/or addition may the one that includes only one type of modification (e.g., substitution), or two or more modifications (e.g., substitution and insertion).

Furthermore, in case of substitution, it is preferable that an amino acid present after substitution is a homologous amino acid to the original amino acid. Herein, amino acids belonging to each of the following groups are designated as homologous amino acids.

(Group 1) Leu, Ile, Val, Met, His, Trp, Tyr, Phe
(Group 2) Glu, Gln, Asp, Asn
(Group 3) Ser, Thr, Cys, Gly, Ala, Pro
(Group 4) Lys, Arg

The number of amino acids subject to substitution, insertion, deletion, and/or addition is not particularly limited, as long as the polypeptide after modification has the enzyme activity to stereoselectively reduce 1-chloro-3-hydroxyacetone to generate (S)-3-chloro-1,2-propanediol. However, sequence identity with the amino acid sequence represented by SEQ ID NO: 1 in the Sequence Listing is preferably 80%. More preferable is 90% or more sequence identity, still more preferable is 95% or more sequence identity, and most preferable is 99% or more sequence identity. Sequence identity is expressed, when the amino acid sequence represented by SEQ ID NO: 1 in the Sequence Listing and a modified amino acid sequence are compared in a similar manner described above to confirm a highly conserved region, by a value obtained by dividing the number of locations where amino acids are identical between the both sequences by the total number of amino acids compared, which is further multiplied by 100.

Any polynucleotide encoding the above polypeptide can be used as a polynucleotide of the present invention. Examples include a polynucleotide comprising the nucleotide sequence represented by SEQ ID NO: 2 in the Sequence Listing, and a polynucleotide that hybridizes with a polynucleotide comprising the nucleotide sequence complementary to the nucleotide sequence represented by SEQ ID NO: 2 in the Sequence Listing under stringent conditions.

Herein, the expression "a polynucleotide that hybridizes with a polynucleotide comprising the nucleotide sequence complementary to the nucleotide sequence represented by SEQ ID NO: 2 in the Sequence Listing under stringent conditions" means a polynucleotide obtained under stringent conditions by using colony hybridization, plaque hybridization, Southern hybridization, or the like with a polynucleotide comprising the nucleotide sequence complementary to the nucleotide sequence represented by SEQ ID NO: 2 in the Sequence Listing working as a probe.

Hybridization can be performed according to the method described in Molecular Cloning, A laboratory manual, second edition (Cold Spring Harbor Laboratory Press, 1989), etc. Herein, the expression "a polynucleotide that hybridizes under stringent conditions" refers to, for example, a polynucleotide that can be obtained, after hybridization is performed in the presence of 0.7 to 1.0 M of NaCl at 65°C by using a filter on which a polynucleotide derived from a colony or a plaque is immobilized, by washing the filter with 2x SSC solution (the SSC solution contains 150 mM sodium chloride and 15 mM sodium citrate) under the conditions of 65°C. A nucleotide can be obtained by washing preferably at 65°C with $0.5\times$ SSC solution, more preferably at 65°C with $0.2\times$ SSC solution, and still more preferably at 65°C with $0.1\times$ SSC solution.

Polynucleotides that can hybridize under the above-described conditions include polypeptides whose sequence identity with the polynucleotide indicated by SEQ ID NO: 2 is 60% or more, preferably 80% or more, more preferably 90% or more, still more preferably 95% or more, and most preferably 99% or more. However, as long as a polypeptide encoded has the activity to stereoselectively reduce 1-chloro-3-hydroxyacetone to generate (S)-3-chloro-1,2-propanediol, such a polynucleotide is included by the polynucleotide of the present invention.

Herein, "sequence identity (%) " is expressed by a value obtained by compared optimally aligning two polynucleotides to be, dividing the number of locations where nucleic acid bases (e.g., A, T, C, G, U, or I) are identical between the both sequences by the total number of bases compared, and multiplying the value thus obtained by 100.

Sequence identity, for example, can be calculated by the following analytical tools: GCG Wisconsin Package (Program Manual for The Wisconsin Package, Version8, Sept. 1994, Genetics Computer Group, 575 Science Drive Medison, Wisconsin, USA 53711; Rice, P. (1996) Program Manual for EGCG Package, Peter Rice, The Sanger Centre, Hinxton Hall, Cambridge, CB10 1RQ, England) ; and the ExPASy World Wide Web molecular biology server (Geneva University Hospital and University of Geneva, Geneva, Switzerland).

An example of the method for obtaining from *Cellulomonas* sp. strain KNK0102 the polypeptide (the enzyme) of the present invention is described below, but the present invention is not limited by this.

First, a microorganism having an enzyme that stereoselectively reduces 1-chloro-3-hydroxyacetone to generate (S)-3-chloro-1,2-propanediol is cultured in a suitable medium. For culturing, as long as the microorganism grows, a common liquid nutrient medium containing a carbon source, anitrogen source, inorganic salts, organic nutrients, etc., can be used. Culture can be performed, for example, at temperature of 25 to 37°C, at pH of 4 to 8, by shaking or aerating.

After microbial cells are separated from the culture solution by centrifugation, the microbial cells are suspended in a suitable buffer, the microbial cells are crushed or dissolved either physically using glass beads, etc., or biochemically with an enzyme, etc., and solids in the solution are removed further by centrifugation, and thereby a crude enzyme solution of the enzyme can be obtained. In addition, the crude enzyme solution can be further purified by a method that those skilled in the art usually employ, for example, by one of ammonium sulfate precipitation, dialysis, chromatography, and the like, or a combination thereof. As for chromatography, one of hydrophobic chromatography, ion-exchange chromatography, and gel-filtration chromatography, or a combination thereof can be also used.

An example of the method for obtaining DNA encoding the enzyme of the present invention is described below, but the present invention is not limited by this.

Primers for PCR (Polymerase Chain Reaction) are synthesized based on the conserved sequence of amino acids commonlypresent among various glycerol dehydrogenases. Then, from a microorganism from which the DNA is derived, chromosomal DNA of the microorganism is prepared by a common method for isolating DNA, e.g., the Hereford method (Cell, 18, 1261 (1979)). PCR is performed using the chromosomal DNA as a template and the above PCR primers, and a part of the DNA encoding the polypeptide (core sequence) is amplified to determine a nucleotide sequence. The nucleotide sequence can be determined by the dideoxy chain termination method, etc. For example, an ABI 373A DNA Sequencer (from Applied Biosystems), etc., can be used.

In order to elucidate the nucleotide sequence in the flanking regions of the core sequence, chromosomal DNA of the microorganism is digested by a restriction enzyme whose recognition sequence is not present in the core sequence. The resulting DNA fragment is self ligated by T4 ligase to prepare a template DNA for inverse PCR (Inverse PCR: Nucleic Acids Res., 16, 8186 (1988)). Then, based on the core sequence, primers that become startingpoints for DNA synthesis extending outward from the core sequence are synthesized, and the flanking regions of the core sequence are amplified by inverse PCR. By elucidating the nucleotide sequence of the resulting DNA, a DNA sequence of the entire encoding region of glycerol dehydrogenase of interest can be known.

As a vector DNA used for transferring DNA of the enzyme of the present invention into a host microorganism, so that the DNA is expressed inside the host microorganism with the DNA transferred therein, any vector DNA able to express the enzyme gene inside a suitable host microorganism can be employed. Such a vector DNA includes, for example, plasmid vectors, phage vectors, cosmid vectors, etc. In addition, shuttle vectors, wherein the gene can be exchanged with another host strain, can be used.

Such a vector contains regulatory elements for operably linked promoters (lacUV5 promoter, trp promoter, trc promoter, tac promoter, lpp promoter, tufB promoter, recA promoter, pL promoter, etc.), and can be suitably used as an expression vector that contains an expression unit operably linked with the DNA of the present invention. For example, pUCNT (WO94/03613), etc., can be preferably used.

The term used herein a "regulatory element" refers to a nucleotide sequence having functional promoters and any related transcription elements (for example, an enhancer, CCAAT box, TATA box, SPI site).

The term used herein "operably linked" refers to that, in order that a gene is expressed, DNA is linked with various regulatory elements that regulate expression thereof, such as promoters and enhancers, in a manner that the regulatory elements can act thereon inside a host microorganism. It is well know by those skilled in the art that the type and kind of regulatory element depend on a host.

Host cells into which a vector having the DNA of the present invention is transferred include bacteria, yeasts, filamentous fungi, plant cells, animal cells, and the like, and E. *coli* is particularly preferable. The DNA of the present invention can be transferred into host cells by the conventional method. When E. *coli* is used as a host cell, the DNA of the present invention can be transferred, for example, by the calcium chloride method.

When, by using the enzyme of the present invention or a recombinant capable of producing the enzyme, a compound having a carbonyl group is stereoselectively reduced to generate an optically active alcohol, NADH is required as coenzyme. By adding to the reaction system a necessary amount of NADH only, reduction may proceed. However, on conducting the reaction, the amount of expensive coenzyme used for the reaction can be significantly reduced, by adding enzyme having an ability to convert the coenzyme that has been oxidized ($NAD^+$) into the reduced form (NADH) (hereinafter referred to as the regeneration ability of coenzyme) together with the substrate thereof, that is, by combining the regeneration system of coenzyme with the enzyme of the present invention. As an enzyme having the regeneration ability of coenzyme, hydrogenase, formate dehydrogenase, alcohol dehydrogenase, glucose-6-phosphate dehydrogenase, glucose dehydrogenase, etc., can be used. Preferably, glucose dehydrogenase and formate dehydrogenase are used. Such a reaction may also be conducted by adding the regeneration system of coenzyme in the asymmetric reduction reaction system, but when a transformant transformed by both the DNA encoding the enzyme of the present invention and the DNA encoding glucose dehydrogenase is used as a catalyst, the reaction can be efficiently conducted without separately preparing the enzyme having the regeneration ability of coenzyme and adding it to the reaction system. Such a transformant can be obtained by incorporating into a single vector the DNA encoding the enzyme of the present invention and the DNA encoding the glucose dehydrogenase, which is then transferred into a host cell, or by incorporating each of these two kinds of DNA into each of two different incompatible vectors separately, which are then transferred into a single host cell.

The production of an optically active alcohol from a compound having a carbonyl group by using the enzyme of the present invention or a culture of the transformant of the present invention is conducted as described below. However, the present invention is not limited by the method below.

First, to a suitable solvent, a compound having a carbonyl group as a substrate, a coenzyme such as $NAD^+$, and a culture of the transformant are added, and while pH is being adjusted, the reaction is allowed to proceed by stirring. This reaction is conducted at temperature from 10 to 70°C, and pH of the reaction solution is kept at 4 to 10 during the reaction. The reaction can be conducted in a batch or continuously. In the case that the reaction is conducted in a batch, the reaction substrate can be added at a charging concentration of from 0.1 to 70 w/v%.

The term used herein a "culture" refers to cells of a microorganism, a culture solution containing microbial cells, or treated microbial cells. Treated microbial cells include, for example, dried microbial cells obtained by dehydration with acetone or diphosphorus pentaoxide or by drying utilizing a desiccator or a fan; a surfactant treated cells; a lytic enzyme treated cells; immobilized microbial cells; cell-free extract preparation, in which microbial cells are crushed; or the like. Furthermore, an enzyme that catalyzes asymmetric reduction can be purified from a culture to provide it for use.

Moreover, for conducting the reaction, when a transformant that produces both the enzyme of the present invention and glucose dehydrogenase is used, the amount of a coenzyme used for the reaction can be significantly reduced by further adding glucose to the reaction system.

The method for collecting optically active alcohols obtained by any of the methods described above is not particularly limited. The alcohols can be, either directly from the reaction solution or after the microbial cells are separated, extracted with a solvent such as ethyl acetate, toluene, t-butyl methyl ether, and hexane, and dehydrated followed by purification by evaporation, crystallization, column chromatography with silica gel, or the like. Thus, highly purified, optically active alcohols can be readily obtained.

[Examples]

Below, the present invention is explained further in detail with examples, but the present invention should not be limited by these examples. In addition, in the description below, "%" means "% by weight" unless otherwise specified.

(Example 1) Cloning of a novel glycerol dehydrogenase

Based on the conserved sequence of amino acids commonly present in glycerol dehydrogenase, a DNA sequence was expected from the amino acid sequence and Primer 1 (5'-ACNGAYGARGGNGMNTTYGA-3': SEQ ID NO: 3) and Primer 2 (5'-GGCATNTKRTGDATNGTYTC-3': SEQ ID NO: 4) are synthesized. A buffer for ExTaq (50 μl) containing: two primers (Primer 1 and Primer 2), each 200 pmol; 1. 2 μg of chromosomal DNA from *Cellulomonas* sp. strain KNK0102; dNTPs, each 10 nmol; and 2.5 Uof ExTaq (fromTakaraShuzo) wasprepared, 30cyclesof thermal denaturation (97°C, 0.5 min.), annealing (45°C, 1 min.), and extension (72°C, 1 min.) were conducted followed by cooling to 4°C, and then amplified DNA was confirmed by agarose gel electrophoresis.

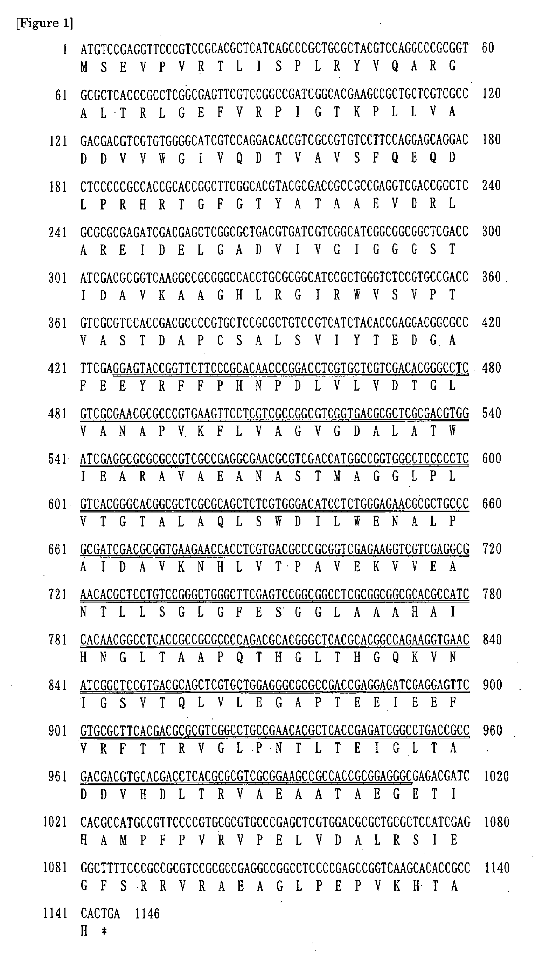

Chromosomal DNA from *Cellulomonas* sp. strain KNK0102 used for this reaction was prepared according to the preparation method in small quantities of bacterial genome DNA described in *Bunshiseibutsugaku Jikken Purotokoru 1* (in Japanese) *(Molecular Biology Experiment Protocols 1)* (Maruzen) p. 36. The amplified DNA was subcloned into a pT7Blue Vector (from Novagen), and the nucleotide sequence thereof was determined. The result showed that the amplified DNA was made of 586 bases excluding the primer sequences. The sequence thereof is a portion of the DNA sequence underlined with double lines in the DNA sequence shown in Figure 1. Hereinaf ter, this sequence is referred to as the "core sequence."

Based on the nucleotide sequence near the 5'-side of the core sequence, the complimentary sequence thereof, Primer 3 (5'-TTCTCCCAGAGGATGTCCCACGAG-3' : SEQ ID NO: 5), was made, and also based on the nucleotide sequence near the 3'-side, Primer 4 (5'-AGATCGAGGAGTTCGTGCGCTTCA-3': SEQ ID NO: 6) was made. For a template for inverse PCR, chromosomal DNA of *Cellulomonas* sp. strain KNK0102 was first digested by the restriction enzyme XmaI, and the digest was self ligated using T4DNA ligase. A buffer for ExTaq (50 µl) containing: 385 ng of this self ligated product; two primers (Primer 3 and Primer 4), each 50 pmol; dNTPs, each 10 nmol; and 2.5 U of ExTaq (from Takara Shuzo) was prepared, 35 cycles of thermal denaturation (97°C, 0.5 min.), annealing (70°C, 1 min.), and extension (72°C, 5 min.) were conducted followed by cooling to 4°C, and then amplified DNA was confirmed by agarose gel electrophoresis.

The amplified DNA was subcloned into a pT7Blue Vector (from Novagen), and the nucleotide sequence thereof was determined. From this result and the result of the core sequence, the entire nucleotide sequence of the gene encoding a glycerol dehydrogenase derived from *Cellulomonas* sp. strain KNK0102 (hereinafter this enzyme is referred to as RCG) was determined. The entire nucleotide sequence and the putative amino acid sequence encoded by the gene are shown together in Figure 1.

(Example 2) Preparation of a recombinant vector comprising the RCG gene

In order that RCG is expressed in E. *coli,* a recombinant vector to be used for transformation was prepared. Double-stranded DNA, in which a NdeI site was added to the initiation codon region of the RCG gene, and a new termination codon and an EcoRI site were added just after the termination codon, was obtained by the method described below.

Based on the nucleotide sequence determined in Example 1, Primer 5 (5'-GATCATATGTCCGAGGTTCCCGTCCGC-3': SEQ ID NO: 7) in which a NdeI site was added to the initiation codon region of the RCG gene, and Primer 6 (5'-CTAGAATTCTTATCAGTGGGCGGTGTGCTTGAC-3' : SEQ ID NO: 8) in which a new termination codon (TAA) and an EcoRI site were added just after the termination codon of the RCG gene were synthesized. A buffer for ExTaq (50 µl) containing: two primers (Primer 5 and Primer 6), each 50 pmol ; 950 ng of chromosomal DNA of *Cellulomonas* sp. strain KNK0102 ; dNTPs, each 10 nmol ; and 2.5 U of ExTaq (fromTakaraShuzo) was prepared, 35 cycles of thermal denaturation (97°C, 0.5 min.), annealing (65°C, 1 min.), and extension (72°C, 1 min.) were conducted followed by cooling to 4°C, and then amplified DNA was confirmed by agarose gel electrophoresis. This amplified fragment was digested by NdeI and EcoRI, which was inserted at NdeI and EcoRI sites downstream of the lac promoter of a plasmid pUCNT (WO94/03613) to obtain the recombinant vector pNTCS.

(Example 3) Addition of a Shine-Dalgarno sequence in the upstream of the RCG gene

In order that the RCG gene is expressed in E. *coli* at a high level, a plasmid in which a Shine-Dalgarno sequence (9 bases) of E. *coli* is additionally added upstream of the initiation codon of the same gene in the plasmid pNTCS prepared in Example 2 was obtained by the method described below.

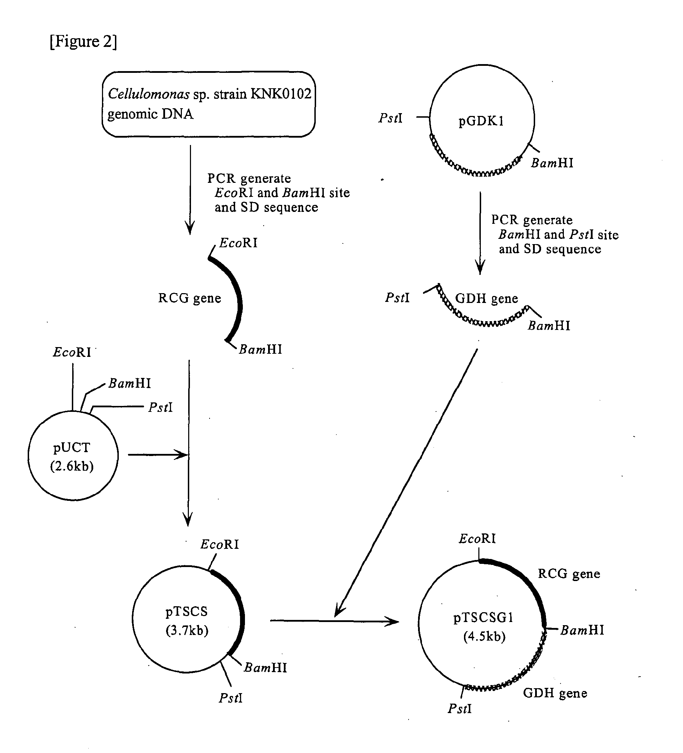

First, G in the NdeI site of the *E. coli* expression vector pUCNT used in Example 2 was converted into T by PCR to construct the plasmid pUCT. Next, based on the nucleotide sequence determined in Example 1, Primer 7 (5'-GCCGAATTCTAAGGAGGTTAACAATGTCCGAGGTTCCCGTCCG-3': SEQ ID NO: 9) in which at 5 bases upstream from the initiation codon of the RCG gene, a Shine-Dalgarno sequence (9 bases) of E. *coli,* and also just before it, an EcoRI site were added; and Primer 8 (5'-GCGGGATCCTTATCAGTGGGCGGTGTGCTTGA-3' : SEQ ID NO: 10) in which just after the termination codon of the RCG gene, a new termination codon (TAA) and a BamHI site were added were synthesized. A buffer for ExTaq (50 µl) containing: two primers (Primer 7 and Primer 8), each 50 pmol; 950 ng of chromosomal DNA of *Cellulomonas* sp. strain KNK0102; dNTPs, each 10 nmol ; and 2.5 U of ExTaq (from Takara Shuzo) was prepared, 35 cycles of thermal denaturation (97°C, 0.5 min.), annealing (65°C, 1 min.), and extension (72°C, 1 min.) were conducted followed by cooling to 4°C, and then amplified DNA was confirmed by agarose gel electrophoresis. This amplified fragment was digested by EcoRI and BamHI, which was inserted at EcoRI and BamHI sites in the downstream of the lac promoter of a plasmid pUCT to obtain the recombinant vector pTSCS. The preparation method and the structure of pTSCS are shown in Figure 2.

(Example 4) Preparation of a recombinant vector simultaneously comprising both of the RCG gene and the glucose dehydrogenase gene.

Double-stranded DNA, in which at 5 bases upstream from the initiation codon of the gene for a glucose dehydrogenase (hereinafter referred to as GDH) derived from *Bacillus megaterium* strain IAM1030, a Shine-Dalgarno sequence (9bases) of E. *coli,* and also just before it, a BamHI site, and just after the termination codon, a PstI site were added, was obtained by the method described below.

Based on the nucleotide sequence information of the GDH gene, Primer 9 (5'-GCCGGATCCTAAGGAGGTTAACAATGTATAAADATTTAGAAGG-3' : SEQ ID NO: 11) in which at 5 bases upstream from the initiation codon of the GDH structural gene, a Shine-Dalgarno sequence (9 bases) of E. *coli,* and also just before it, a BamHI site were added, and Primer 10 (5'-GCGCTGCAGTTATCCGCGTCCTGCTTGGA-3': SEQ ID NO: 12) in which just after the termination codon, a PstI site was added were synthesized by the conventional method. By using these two primers, a plasmid pGDK1 *(*Eur. J. Biochem. , 186, 389 (1989)) being a template, double-stranded DNA was synthesized by PCR. The resulting DNA fragment was digested by BamHI and PstI, which was inserted at the BamHI-PstI sites of pTSCS constructed in Example 3 to obtain the recombinant vector pTSCSG1. The preparation method and the structure of pTSCSG1 are shown in Figure 2.

(Example 5) Preparation of recombinant E. *coli*

By using the recombinant vector pTSCS obtained in Example 3 and the recombinant vector pTSCSG1 obtained in Example 4, E. *coli* HB101 (from Takara Shuzo) was transformed to obtain recombinant E. *coli* HB101 (pTSCS ) and recombinant E. *coli* HB101 (pTSCSG1), respectively. The resulting recombinant, E. *coli* HB101 (pTSCS), was deposited at International Patent Organism Depositary of National Institute of Advanced Industrial Science and Technology on May 12, 2004, Deposit number being FERM BP-10024.

(Example 6) Expression of RCG in recombinant E. *coli*

Each of the recombinant E. *coli* HB101 (pTSCS) and HB101 (pTSCSG1) obtained in Example 5, and E. *coli* HB101 (pUCT), a transformant containing a simple vector plasmid, was cultured in a 2xYT medium (1.6% of Bactotrypton, 1.0% Bactoyeast extract, 0.5% NaCl; pH 7.0) containing 100 $\mu$g/ml of ampicillin. Microorganisms were collected, and then suspended in a 100 mM phosphate buffer (pH 6.5), which was disrupted by ultrasonication using a UH-50 ultrasonic homogenizer (from SMT) to obtain a cell-free extract. The RCG activity of this cell-free extract was determined by adding 20 mM of the substrate 1-chloro-3-hydroxyacetone, 0.167 mM of coenzyme NADH, and an enzyme solution to a 100 mM phosphate buffer (pH 6.5) followed by the measurement of a decrease in absorbance at the wavelength of 340 nm at 30°C. The enzyme activity to oxidize 1 $\mu$mol of NADH to NAD$^+$ for a minute under these reaction conditions was defined as 1 unit.

Moreover, the GDH activity was determined by adding 0.1 M of the substrate glucose, 2 mM of coenzyme NADP$^+$, and an enzyme to a 1 M Tris-HCl buffer (pH 8.0) followed by the measurement of an increase in absorbance at the wavelength of 340 mM at 25°C. The enzyme activity to reduce 1 $\mu$mol of NADP$^+$ to NADPH for a minute under these reaction conditions was defined as 1 unit.

As shown in Table 1, with E. *coli* HB101 (pTSCS) and with HB101 (pTSCSG1), an increase in RCG activity was observed as compared with *E. coli* HB101 (pUCT), a transformant containing a simple vector plasmid.

[Table 1]

| Strain | Relative activity of the CPD oxidation (U/mg) | GDH relative activity (U/mg) |
|---|---|---|
| HB101 (pUCT) | <0.1 | <0.01 |
| HB101 (pTSCS) | 23.5 | <0.01 |
| HB101 (pTSCSG1) | 16.3 | 90.5 |

(Example 7) Purification of RCG from recombinant E. *coli* HB101 (pTSCS)

Into shaking flasks, 60 ml of a liquid medium (pH 7.0) containing 1.6% of Bacto trypton, 1. 0% of Bacto yeast extract, and 0.5% of NaCl was divide followed by steam pasteurization at 120°C for 20 min. To this liquid medium, ampicillin was added at the final concentration of 100 μg/ml, and recombinant *E. coli* HB101 (pTSCS) was inoculated followed by shake culture at 37°C for 3 0 hrs . From 60 ml of the resulting culture solution, microbial cells were collected by centrifugation, which were washed with 120 ml of a 10 mM phosphate buffer (pH 7.0) , and suspended in 60 ml of a 10 mM phosphate buffer (pH 7.0) . Then, the microbial cells were disrupted by ultrasonication using a SONIFIRE 250 (from BRANSON), and residue of the microbial cells were removed by centrifugation to obtain 60 ml of a cell-free extract. This was provided to a TOYOPEARL Super-Q 650S column (from Tosoh) equilibrated in advance with a 10 mM phosphate buffer (pH 7.0) and the enzyme was adsorbed thereon, which was then eluted with linear gradient of NaCl (from 0 mM to 400 mM) to obtain a fraction having an activity to reduce 1-chloro-3-hydroxyacetone. By this purification process, a purified enzyme preparation, which was singular in terms of electrophoresis, was obtained. The molecular weight of the band on SDS electrophoresis was about 43,000.

(Example 8) Determination of characteristics of RCG

The enzyme chemical characteristics of the resulting RCG were studied.

(1) The action

The enzyme acted on 1-chloro-3-hydroxyacetone using NADH as a coenzyme to reduce it into (S)-3-chloro-1,2-propanediol having optical purity of 99.3% e. e.

(2) The molecular weight

By using a 50 mM phosphate buffer (pH 7.0) containing 150 mM NaCl as an eluent, the purified enzyme was analyzed by gel-filtration chromatography using Superdex 200 HR 10/30 (from Pharmacia Biotech). As a result, by calculating from relative retention times of standard proteins, the molecular weight of the enzyme was about 340,000.

(3) The optimum temperature In order to study the optimum temperature of RCG, the activity to oxidize glycerol was determined at 10 to 70°C. The activity was determined by adding 0.1 M of the substrate glycerol, 2 mM of coenzyme $NAD^+$, and the enzyme solution to a 100 mM phosphate buffer (pH 8.0) to react at 10 to 70°C for 3 min. followed by the measurement of an increase in absorbance at 340 mM. The results showed that the optimum temperature was 60 to 70°C.

(4) The optimum pH for reduction

In order to study the optimum pH of RCG for reduction, the activity to reduce dihydroxyacetone at pH 4 to pH 9 was determined. The enzyme activity was determined by adding 10 mM of the substrate dihydroxyacetone, 0.167 mM of coenzyme NADH, 50 mM of KCl, 50 mM of $NH_4Cl$, and the enzyme solution to a buffer to react at 30°C for 3 min. followed by the measurement of a decrease in absorbance at 340 mM. As buffers, 100 mM acetic acid buffers (pH 4.0 to pH 6.0) , 100 mM phosphate buffers (pH 6.0 to pH 8.0), and 100 mM Tris-HCl buffers (pH 8.0 to 9.0) were used to measure the activity at pH 4 to pH 9. The results showed that the optimum pH for reduction was pH 6.0.

(5) The optimum pH for oxidation

In order to study the optimum pH of RCG for oxidation, the activity to oxidize glycerol at pH 6 to pH 11 was determined. The enzyme activity was determined by adding 0.1 M of the substrate glycerol, 2 mM of coenzyme $NAD^+$, 50 mM of KCl, 50 mM of $NH_4Cl$, and the enzyme solution to a buffer to react at 30°C for 3 min. followed by the measurement of an increase in absorbance at 340 mM. As buffers, 100 mM phosphate buffers (pH 6.0 to pH 8.0), 100 mM Tris-HCl buffers (pH 8.0 to 9.0), and 100 mM carbonic acid buffers (pH 9.0 to pH 11.0) were used to measure the activity at pH 6 to pH 11. The results showed that the optimum pH for oxidation was pH 9.0.

(Example 9) Substrate specificity of RCG

Oxidation activity was determined by adding 2 mM of coenzyme $NAD^+$, 10 mM of a compound shown in Table 2, and the enzyme solution to a 100 mM phosphate buffer (pH 8.0) to react at 30°C for 3 min. followed by the measurement of an increase in absorbance at the wavelength of 340 mM. In Table 2, relative activities are summarized, wherein the activity for glycerol is 100.

[Table 2]

| Substrate (10 mM) | Relative activity (%) |
| --- | --- |
| glycerol | 100 |
| ethylene glycol | 8 |
| 1,2-propanediol | 142 |
| 1,3-propanediol | 1 |
| 3-methoxy-1,2-propanediol | 39 |
| 3-mercapto-1,2-propanediol | 129 |

(continued)

| Substrate (10 mM) | Relative activity (%) |
|---|---|
| 3-methylthio-1,2-propanediol | 120 |
| 3-chloro-1,2-propanediol | 63 |
| 3-bromo-1,2-propanediol | 58 |
| 3-phenoxy-1,2-propanediol | 9 |
| 1,2-butanediol | 129 |
| 1,3-butanediol | 3 |
| 1,4-butanediol | 1 |
| 2,3-butanediol | 64 |
| 1,2-pentanediol | 153 |
| 1,4-pentanediol | 0 |
| 1,2-hexanediol | 26 |
| methanol | 0 |
| ethanol | 0 |
| propanol | 0 |
| isopropanol | 0 |
| 1-amino-2-propanol | 12 |
| 1,3-dichloro-2-propanol | 0 |
| butanol | 0 |
| 2-butanol | 0 |
| 1,2,4-butanetriol | 21 |

(Example 10) Synthesis of (S)-3-chloro-1,2-propanediol from 1-chloro-3-hydroxyacetone by using recombinant E. *coli* with the RCG gene transferred therein

To 1 ml of a cell-free extract of recombinant E. *coli* HB101 (pTSCS) obtained in Example 6, 100 units of glucose dehydrogenase (from Amano Enzyme), 10 mg of 1-chloro-3-hydroxyacetone, 1 mg of $NAD^+$, and 20 mg of glucose were added to shake at 30°C for 5 hrs. After the reaction was completed, the reaction solution was saturated with ammonium sulfate, and ethyl acetate was added to perform extraction. The resulting (S)-3-chloro-1,2-propanediol was analyzed by capillary gas chromatography.

[Conditions for analysis]

Column: HP-5; 30 m by 0.32 mm I. D. (from Agilent Technologies);
Detection: FID;
Initial column temperature: 50°C; final column temperature: 200°C; temperature raising rate: 6°C/min.;
Charging temperature: 150°C;
Detection temperature: 300°C;
Carrier gas: helium (70 kPa);
Split ratio: 100/1; and
Detection time: 1-chloro-3-hydroxyacetone: 8.2 min.; 3-chloro-1,2-propanediol: 10.2 min.
Furthermore, optical purity of the resulting 3-chloro-1,2-propanediol was determined by high performance liquid chromatography after tosylation.

[Conditions for analysis]

Column: Chiralpak AD (from Daicel Chemical Industries) ;
Eluent: hexane/isopropanol = 9/1;
Flow rate: 0.8 ml/min.;
Detection: 235 nm;
Column temperature: 30°C;
Elution time: S-stereoisomer: 29 min.; R-stereoisomer: 34 min.; and

$$Optical\ purity\ (\%\ ee) = (A - B)/(A + B) \times 100$$

(A and B refer to amounts of corresponding enantiomers, and A is greater than B).

The results showed that the amount of (S)-3-chloro-1,2-propanediol generated was 7.6 mg; and optical purity was 99.4% ee.

(Example 11) Synthesis of (S)-3-chloro-1,2-propanediol from 1-chloro-3-hydroxyacetone by using recombinant E. *coli* with RCG and a glucose dehydrogenase simultaneously expressed therein

To 50 ml of a cell-free extract of recombinant E. *coli* HB101 (pTSCSG1) obtained in Example 6, 10 g of glucose, 5 mg of NAD, and 5 g of 1-chloro-3-hydroxyacetone were added. This reaction solution was stirred at 30°C for 24 hrs, while pH was being adjusted to 6.5 by dropping 5 M NaOH. After the reaction was completed, the reaction solution was saturated with ammonium sulfate, and ethyl acetate was added to perform extraction followed by the analysis similarly to Example 10. The results showed that the amount of (S)-3-chloro-12-propanediol generated was 4.3 g ; and optical purity was 99.3% ee.

(Example 12) Evaluation of the stability of RCG to 1-chloro-3-hydroxyacetone and to 3-chloro-1,2-propanediol

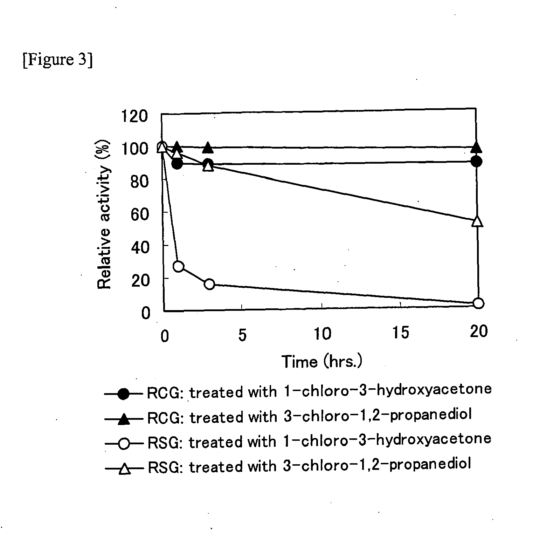

In order to study the stability of RCG to 1-chloro-3-hydroxyacetone and to 3-chloro-1,2-propanediol, 1-chloro-3-hydroxyacetone was added at the final concentration of 0.25% to the enzyme solution and incubated at 30°C followed by the measurement of the activity to reduce 1-chloro-3-hydroxyacetone by using the above enzyme solution. Also, 3-chloro-1,2-propanediol was added at the final concentration of 1% to the enzyme solution and incubated at 30°C followed by the measurement of the activity to reduce 1-chloro-3-hydroxyacetone. As shown in Figure 3, the results showed that as compared with a glycerol dehydrogenase RSG (WO03/018523) derived from *Serratia marcescens,* whichisknown to stereoselectively reduce 1-chloro-3-hydroxyacetone into an S-stereoisomer, RCG was very stable to 1-chloro-3-hydroxyacetone and to 3-chloro-1,2-propanediol.

| 0-1 | Form PCT/RO/134 (SAFE) The indications relating to the deposited microorganisms or | |
|---|---|---|
| 0-1-1 | other biological material (PCT Rule 13 bis) were made by the following. | JPO-PAS 0324 |
| 0-2 | International application No. | PCT/JP2005/010960 |
| 0-3 | Applicant's or agent's file reference | B040280WO01- |

| 1 | The indications made below relate to the microorganism or biological material referred to in the detailed description of the invention. | |
|---|---|---|
| 1-1 | Paragraph Number | 0073 |
| 1-3 | Indications of deposit | |
| 1-3-1 | Name of depositary institution | International Patent Organism Depositary (IPOD), National Institute of Advanced Industrial Science and Technology |
| 1-3-2 | Address of depositary institution | Tsukuba Central 6, 1-1-1 Higashi, Tsukuba, Ibaraki 305-8566, Japan |
| 1-3-3 | Date of deposit | December 05, 2004 (12. 05. 2004) |
| 1-3-4 | Accession Number | IPOD FERM BP-10024 |
| 1-5 | Designated States for which indications are made | all designated States |
| For receiving Office use only | | |
| 0-4 | This sheet was received with the international application (Yes/No) | yes |

(continued)

| For receiving Office use only | | |
|---|---|---|
| 0-4-1 | Authorized officer | Misako Akutsu |
| For International Bureau use only | | |
| 0-5 | This sheet was received by the International Bureau on: | |
| 0-5-1 | Authorized officer | |

**Claims**

1.  A polypeptide having physicochemical characteristics of (1) to (5) described below:

    (1) action: act on 1-chloro-3-hydroxyacetone using NADH as a coenzyme to generate (S)-3-chloro-1,2-propanediol ;
    (2) molecular weight: about 340,000 by gel-filtration and about 43,000 by SDS polyacrylamide gel electrophoresis;
    (3) optimum temperature: from 60 to 70°C;
    (4) optimum pH for reduction: 6.0; and

(5) optimum pH for oxidation: 9.0.

2. The polypeptide set forth in Claim 1, which is resistant to 1-chloro-3-hydroxyacetone.

3. The polypeptide set forth in Claim 1 or Claim 2, wherein the polypeptide is derived from *Cellulomonas* sp. strain KNK0102.

4. A polypeptide (a) or (b) described below:

   (a) a polypeptide comprising an amino acid sequence represented by SEQ ID NO: 1 in the Sequence Listing; or
   (b) a polypeptide comprising an amino acid sequence in which one or more amino acids are substituted, inserted, deleted, and/or added in the amino acid sequence represented by SEQ ID NO: 1 in the Sequence Listing, and having an enzyme activity to stereoselectively reduce 1-chloro-3-hydroxyacetone to generate (S)-3-chloro-1,2-propanediol.

5. A DNA encoding the polypeptide set forth in any one of Claims 1 to 4.

6. A DNA (a) or (b) described below:

   (a) a DNA comprising a nucleotide sequence represented by SEQ ID NO: 2 in the Sequence Listing; or
   (b) a DNA that hybridizes with a DNA comprising a nucleotide sequence complementary to the nucleotide sequence represented by SEQ ID NO: 2 in the Sequence Listing under stringent conditions and that encodes a polypeptide having an enzyme activity to stereoselectively reduce 1-chloro-3-hydroxyacetone to generate (S)-3-chloro-1,2-propanediol.

7. An expression vector containing the DNA set forth in Claim 5 or Claim 6.

8. The expression vector set forth in Claim 7, wherein the expression vector is the plasmid pTSCS shown in Figure 2.

9. A transformant obtained by transforming a host cell by using the expression vector set forth in Claim 7 or Claim 8.

10. The transformant set forth in Claim 9, wherein said host cell is *Escherichia coli.*

11. The transformant set forth in Claim 10, wherein the *Escherichia coli* is *Escherichia coli* HB101 (pTSCS) (FERM BP-10024).

12. A manufacturing method of an optically active alcohol **characterized by** reacting the polypeptide set forth in any one of Claims 1 to 4 or a culture of the transformant set forth in any one of Claims 9 to 11 with a compound having a carbonyl group.

13. The manufacturing method set forth in Claim 12, wherein (S)-3-chloro-1,2-propanediol is manufactured from 1-chloro-3-hydroxyacetone.

[Figure 1]

```
   1 ATGTCCGAGGTTCCCGTCCGCACGCTCATCAGCCCGCTGCGCTACGTCCAGGCCCGCGGT   60
     M  S  E  V  P  V  R  T  L  I  S  P  L  R  Y  V  Q  A  R  G

  61 GCGCTCACCCGCCTCGGCGAGTTCGTCCGGCCGATCGGCACGAAGCCGCTGCTCGTCGCC  120
     A  L  T  R  L  G  E  F  V  R  P  I  G  T  K  P  L  L  V  A

 121 GACGACGTCGTGTGGGGCATCGTCCAGGACACCGTCGCCGTGTCCTTCCAGGAGCAGGAC  180
     D  D  V  V  W  G  I  V  Q  D  T  V  A  V  S  F  Q  E  Q  D

 181 CTCCCCCGCCACCGCACCGGCTTCGGCACGTACGCGACCGCCGCCGAGGTCGACCGGCTC  240
     L  P  R  H  R  T  G  F  G  T  Y  A  T  A  A  E  V  D  R  L

 241 GCGCGCGAGATCGACGAGCTCGGCGCTGACGTGATCGTCGGCATCGGCGGCGGCTCGACC  300
     A  R  E  I  D  E  L  G  A  D  V  I  V  G  I  G  G  G  S  T

 301 ATCGACGCGGTCAAGGCCGCGGGCCACCTGCGCGGCATCCGCTGGGTCTCCGTGCCGACC  360
     I  D  A  V  K  A  A  G  H  L  R  G  I  R  W  V  S  V  P  T

 361 GTCGCGTCCACCGACGCCCCGTGCTCCGCGCTGTCCGTCATCTACACCGAGGACGGCGCC  420
     V  A  S  T  D  A  P  C  S  A  L  S  V  I  Y  T  E  D  G  A

 421 TTCGAGGAGTACCGGTTCTTCCCGCACAACCCGGACCTCGTGCTCGTCGACACGGGCCTC  480
     F  E  E  Y  R  F  F  P  H  N  P  D  L  V  L  V  D  T  G  L

 481 GTCGCGAACGCGCCCGTGAAGTTCCTCGTCGCCGGCGTCGGTGACGCGCTCGCGACGTGG  540
     V  A  N  A  P  V  K  F  L  V  A  G  V  G  D  A  L  A  T  W

 541 ATCGAGGCGCGCGCCGTCGCCGAGGCGAACGCGTCGACCATGGCCGGTGGCCTCCCCCTC  600
     I  E  A  R  A  V  A  E  A  N  A  S  T  M  A  G  G  L  P  L

 601 GTCACGGGCACGGCGCTCGCGCAGCTCTCGTGGGACATCCTCTGGGAGAACGCGCTGCCC  660
     V  T  G  T  A  L  A  Q  L  S  W  D  I  L  W  E  N  A  L  P

 661 GCGATCGACGCGGTGAAGAACCACCTCGTGACGCCCGCGGTCGAGAAGGTCGTCGAGGCG  720
     A  I  D  A  V  K  N  H  L  V  T  P  A  V  E  K  V  V  E  A

 721 AACACGCTCCTGTCCGGGCTGGGCTTCGAGTCCGGCGGCCTCGCGGCGGCGCACGCCATC  780
     N  T  L  L  S  G  L  G  F  E  S  G  G  L  A  A  A  H  A  I

 781 CACAACGGCCTCACCGCCGCGCCCCAGACGCACGGGCTCACGCACGGCCAGAAGGTGAAC  840
     H  N  G  L  T  A  A  P  Q  T  H  G  L  T  H  G  Q  K  V  N

 841 ATCGGCTCCGTGACGCAGCTCGTGCTGGAGGGCGCGCCGACCGAGGAGATCGAGGAGTTC  900
     I  G  S  V  T  Q  L  V  L  E  G  A  P  T  E  E  I  E  E  F

 901 GTGCGCTTCACGACGCGCGTCGGCCTGCCGAACACGCTCACCGAGATCGGCCTGACCGCC  960
     V  R  F  T  T  R  V  G  L  P  N  T  L  T  E  I  G  L  T  A

 961 GACGACGTGCACGACCTCACGCGCGTCGCGGAAGCCGCCACCGCGGAGGGCGAGACGATC 1020
     D  D  V  H  D  L  T  R  V  A  E  A  A  T  A  E  G  E  T  I

1021 CACGCCATGCCGTTCCCCGTGCGCGTGCCCGAGCTCGTGGACGCGCTGCGCTCCATCGAG 1080
     H  A  M  P  F  P  V  R  V  P  E  L  V  D  A  L  R  S  I  E

1081 GGCTTTTCCCGCCGCGTCCGCGCCGAGGCCGGCCTCCCCGAGCCGGTCAAGCACACCGCC 1140
     G  F  S  R  R  V  R  A  E  A  G  L  P  E  P  V  K  H  T  A

1141 CACTGA 1146
     H  *
```

[Figure 2]

[Figure 3]

—●— RCG: treated with 1-chloro-3-hydroxyacetone
—▲— RCG: treated with 3-chloro-1,2-propanediol
—○— RSG: treated with 1-chloro-3-hydroxyacetone
—△— RSG: treated with 3-chloro-1,2-propanediol

<table>
<tr><td colspan="2">INTERNATIONAL SEARCH REPORT</td><td>International application No.<br>PCT/JP2005/010960</td></tr>
</table>

| A. CLASSIFICATION OF SUBJECT MATTER |
|---|
| Int.Cl$^7$ C12N15/53, 1/21, 9/04, C12P7/18 |

According to International Patent Classification (IPC) or to both national classification and IPC

| B. FIELDS SEARCHED |
|---|
| Minimum documentation searched (classification system followed by classification symbols)<br>    Int.Cl$^7$ C12N15/53, 1/21, 9/04, C12P7/18 |

Documentation searched other than minimum documentation to the extent that such documents are included in the fields searched

| Jitsuyo Shinan Koho | 1922-1996 | Jitsuyo Shinan Toroku Koho | 1996-2005 |
| Kokai Jitsuyo Shinan Koho | 1971-2005 | Toroku Jitsuyo Shinan Koho | 1994-2005 |

Electronic data base consulted during the international search (name of data base and, where practicable, search terms used)
GenBank/EMBL/DDBJ/GeneSeq, SwissProt/PIR/Geneseq, CAplus/BIOSIS/MEDLINE/ WPIDS(STN)

| C. DOCUMENTS CONSIDERED TO BE RELEVANT | | |
|---|---|---|
| Category* | Citation of document, with indication, where appropriate, of the relevant passages | Relevant to claim No. |
| X<br>Y | WO 2003/018523 A1 (KANEKA Corp.),<br>03 June, 2003 (03.06.03),<br>& EP 1422213 A1      & US 2004/0214881 A1 | 1-3,12,13<br>1-13 |
| Y | WO 98/37204 A1 (WISCONSIN ALUMNI RESEARCH FOUNDATION),<br>27 August, 1998 (27.08.98),<br>& US 6087140 A | 1-13 |
| Y | MALLINDER, P.R. et al., Cloning and characterization of a gene from Bacilus stearothermophilus var.non-diastaticus encoding a glycerol dehydrogenase., Gene (1992), Vol.110, No.1, pages 9 to 16 | 1-13 |

| ☒ Further documents are listed in the continuation of Box C. | ☐ See patent family annex. |
|---|---|

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search<br>    18 August, 2005 (18.18.05) | Date of mailing of the international search report<br>    06 September, 2005 (06.09.05) |
|---|---|
| Name and mailing address of the ISA/<br>    Japanese Patent Office | Authorized officer |
| Facsimile No. | Telephone No. |

Form PCT/ISA/210 (second sheet) (January 2004)

**INTERNATIONAL SEARCH REPORT**

| International application No. |
|---|
| PCT/JP2005/010960 |

C (Continuation).    DOCUMENTS CONSIDERED TO BE RELEVANT

| Category* | Citation of document, with indication, where appropriate, of the relevant passages | Relevant to claim No. |
|---|---|---|
| Y | DANIEL, R. et al., Biochemical and molecular characterization of the oxidative branch of glycerol utilization by Citrobacter freundii, J.Bacteriol., (1995), Vol.177, No.15, pages 4392 to 4401 | 1-13 |
| Y | JP 2003-061668 A  (KANEKA Corp.), 04 March, 2003 (04.03.03), (Family: none) | 1-13 |
| Y | FONG, K.P.Y. et al., Characterization and expression of the plasmid-borne bedD gene from Pseudomonas putida ML2, which codes for a $NAD^+$-dependent cis-benzene dihydrodiol dehydrogenase., J.Bacteriol.(1996), Vol.178, No.19, pages 5592 to 5601 | 1-13 |

Form PCT/ISA/210 (continuation of second sheet) (January 2004)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 10113170 A
- WO 03018523 A
- JP 2003061668 A
- WO 9403613 A
- JP 2005010960 W

**Non-patent literature cited in the description**

- *J. Gen. Microbiol.,* 1984, vol. 130, 3225
- *J. Gen. Microbiol.,* 1985, vol. 131, 1581
- *J. Gen. Microbiol.,* 1990, vol. 136, 1043
- *Biochim. Biochem. Acta,* 1989, vol. 994, 270
- *Agric. Biol. Chem.,* 1982, vol. 48, 1603
- *J. Bacteriol.,* 1979, vol. 140, 182
- Current Protocols in MolecularBiology. JohnWiley-andSons, Inc, 1989
- Molecular Cloning, A laboratory manual. Cold Spring Harbor Laboratory Press, 1989
- *Cell,* 1979, vol. 18, 1261
- *Nucleic Acids Res.,* 1988, vol. 16, 8186
- *Eur. J. Biochem.,* 1989, vol. 186, 389